Europäisches Patentamt

**European Patent Office**

Office européen des brevets

(19)

(11) Numéro de publication: **0 236 209**
**A1**

---

(12) ## DEMANDE DE BREVET EUROPEEN

(21) Numéro de dépôt: **87400354.4**

(51) Int. Cl.⁴: **C 12 N 15/00,** C 12 N 9/64

(22) Date de dépôt: **19.02.87**

---

(30) Priorité: **21.02.86 FR 8602380**

(43) Date de publication de la demande: **09.09.87**
**Bulletin 87/37**

(84) Etats contractants désignés: **AT BE CH DE FR GB IT LI
LU NL SE**

(71) Demandeur: **GENETICA, 160 Quai de Polangis,
94340 Joinville Le Pont (FR)**

(72) Inventeur: **Caillaud, Frédéric, 78 Boulevard des
Etats-Unis, F-78110 le Vesinet (FR)**
Inventeur: **Latta, Martine, 297 Rue de Charenton 75,
Paris 12ème (FR)**
Inventeur: **Mayaux, Jean-François, 2iter Boulevard de la
République, F-92260 Fontenay aux Roses (FR)**
Inventeur: **Sarmientos, Paolo, Via Mose Bianchi 104,
Milano (IT)**

(74) Mandataire: **Pilard, Jacques et al, RHONE-POULENC
INTERSERVICES Service Brevets Pharma 25, Quai Paul
Doumer, F-92408 Courbevoie Cédex (FR)**

(54) **Préparation par voie microbiologique de l'activateur tissulaire humain du plasminogène (t-PA) et conversion de l'enzyme
ainsi obtenue en sa forme active.**

(57) Procédé de préparation de l'activateur tissulaire humain du
plasminogène (t-PA) par culture d'une bactérie (E. coli) capable
d'assurer le maintien stable d'un plasmide contenant le promoteur P_L du bactériophage lambda ou le promoteur Ptrp de l'opéron tryptophane d'E. coli, le site de fixation des ribosomes du
gène cII du bactériophage lambda et le codon d'initiation ATG
fusionné avec le gène de structure de l'activateur humain du
plasminogène et la conversion de l'enzyme ainsi obtenue en sa
forme active.

EP 0 236 209 A1

La présente invention concerne un procédé de préparation par voie microbiologique d'une enzyme humaine et sa conversion en sa forme active.

Plus particulièrement, l'invention concerne un procédé, mettant en oeuvre les techniques de manipulations génétiques in vitro, pour produire "l'activateur tissulaire humain du plasminogène" ("t-PA") dans un microorganisme tel qu'Escherichia coli ainsi qu'un procédé, mettant en oeuvre des méthodes chimiques de dénaturation et renaturation, pour convertir l'enzyme ainsi obtenue en sa forme active.

Une cause principale de mortalité humaine dans les pays développés est le blocage de vaisseaux sanguins par la formation de caillots (phénomène de thrombose). Dans le plasma des mammifères la synthèse et la dégradation des caillots sanguins relèvent de mécanismes très finement controlés auxquels plusieurs enzymes participent. La synthèse du caillot est un processus nécessaire pour éviter les hémorragies. Une fois une hémorragie stoppée, le caillot doit être ensuite dégradé pour revenir à une circulation sanguine normale. La dégradation des caillots, composés principalement de fibrine, est normalement effectuée par une puissante enzyme fibrinolytique, la plasmine qui dérive d'un précurseur inactif, le plasminogène. Le rôle du t-PA est la protéolyse limitée du zymogène (plasminogène) pour donner l'enzyme active (plasmine) [T. Astrup dans E. Reich, D.B. Rifkin et E. Shaw (ed), Proteases and Biological Control, Cold Spring Harbor Laboratory Press, NY, p. 343 et suivantes (1975) ; J.K. Christman et coll. dans A.J. Barret (ed), Proteinases in Mammalian Cells and Tissues, Elsevier/North Holland Biomedical Press, NY, p. 91 et suivantes (1977), E. Reich dans R.D. Berlin, H. Herman, J.H. Lepow et J.M. et J.M. Tauzer (ed), Molecular Basis of Biological Degradative Processes, Academic Press, NY, p. 155 et suivantes (1978) ; D. Collen, Thrombosis Haemostasis 43, p. 77 et suivantes (1980)].

On sait isoler du t-PA à partir de diverses sources telles le sang, l'utérus humain, le coeur du porc et des cultures de cellules tumorales [S.S. Husain et coll., Proc. Nat. Acad. Sci. USA 78, p. 4264 et suivantes (1981) ; D.C. Rijken et coll., Biochim. Biophys. Acta 580, p. 140 et suivantes (1979) ; E.R. Cole et F.W. Bachmann, J. Biol. Chem. 252, p. 3729 et suivantes (1977) ; D.C. Rijken et D. Collen, J. Biol. Chem. 256, p. 7035 et suivantes (1981)].

Le t-PA a été comparé à un autre activateur du plasminogène, l'urokinase : il possède des propriétés immunologiques différentes ainsi qu'une forte affinité pour la fibrine conduisant à une augmentation de son activité [D.C. Rijken et coll., J. Biol. Chem. 257, p. 2920 et suivantes (1982) ; M. Ranby et coll., Thromb. Res. 27, p. 175 et suivantes (1982)]. Ces caractéristiques du t-PA lui confèrent des avantages thérapeutiques considérables car la lyse générale du fibrinogène est improbable et les risques d'hémorragie sont limités.

Plusieurs lignées cellulaires naturelles, productrices de t-PA, ont été décrites dans la littérature comme moyen d'accès au t-PA (D.C. Rijken et D. Collen, supra, ; F.L. Wilson et coll., Cancer Res 40, p. 933 et suivantes (1980) ; M. Gronow et R. Bliem, Trends Biotech, 1, p. 26 et suivantes (1983) ; D. Vetterlein et coll., J. Biol. Chem. 255, p. 3665 et suivantes (1980) ; W. Weimar et coll., The Lancet 2, p. 1018 et suivantes (1981)), mais aucune d'entre elles ne conduit à un niveau de production de t-PA qui puisse répondre aux besoins d'un marché mondial potentiel. De plus, parmi ces sources naturelles, les meilleures lignées cellulaires productrices sont d'origine tumorale.

Les techniques de recombinaison génétique _in vitro_ offrent maintenant la possibilité de faire synthétiser par un microorganisme, par exemple la bactérie _Escherichia coli_, n'importe quelle protéine ou n'importe quel polypeptide, et ce théoriquement en quantités illimitées (voir par exemple F. Gros et coll., Sciences de la Vie et Société, Documentation Française éd. 1979)

On sait, depuis les expériences classiques de F. Jacob et J. Monod, que l'ADN contient d'une part un ensemble de gènes dits "de structure", c'est-à-dire codant pour une protéine donnée, et d'autre part des gènes dits "de régulation", c'est à dire capable de moduler l'expression des gènes de structure, l'association des deux types formant une entité dite "opéron".

Les recherches en biologie moléculaire et la mise au point des techniques de séquençage de l'ADN (F. Sanger et A.R. Coulson, J. Mol. Biol., 94, p. 441 et suivantes (1975), A.M. Maxam et W. Gilbert, Proc. Natl. Acad. Sci. (USA) 74, p. 560 et suivantes (1977)), ont permis de préciser l'organisation de l'opéron telle que l'avaient conçue F. Jacob et J. Monod (F. Jacob et J. Monod, Cold Spring Harbour Symp. Quant. Biol, 26, p. 193 et suivantes (1961), F. Jacob et J. Monod, J. Mol. Biol. 3, p. 318 et suivantes (1961)), et d'identifier les caractères particuliers de la structure primaire des deux types de gènes.

Ainsi, tout gène de structure est encadré d'un codon dit "d'initiation de traduction"(ATG) et d'un codon "d'arrêt". Le codon d'initiation a pour fonction de fixer un ARN de transfert initiateur porteur d'une formylméthionine. La chaîne protéique s'allongera à partir de cette formylméthionine par attachements successifs des acides aminés codés par le gène de structure, le codon "d'arrêt" provoquera enfin un arrêt de l'allongement et la libération de la protéine néoformée.

En ce qui concerne les gènes régulateurs (promoteurs, répresseurs), définissant par exemple un promoteur comme un fragment d'ADN sur lequel se fixe l'ARN polymérase, on a pu identifier les séquences d'ADN les plus conservées ; de même on a pu définir les séquences d'ADN les plus conservées au niveau des sites de fixation de ribosomes (RBS) (J.Shine et L. Dalgarno, Nature, 254, p. 34 et suivantes (1975)), sites qui jouent un rôle dans la traduction en protéine de l'ARN messager.

Les gènes régulateurs bactériens peuvent donc être définis par leur propriétés fonctionnelles ainsi que par leur séquence primaire, ce dont les techniques de recombinaisons génétiques in vitro tirent profit pour placer un gène de structure quelconque sous leur contrôle, ceci grâce à l'existence des "enzymes de restriction", qui coupent l'ADN en des points spécifiques (H.O. Smith et K.W. Wilcox, J. Mol. Biol. 51, p. 379 et suivantes (1970), M. Meselson et R. Yuan, Nature, 217, p. 1110 et suivantes (1968), R.J. Roberts, Nucleic Acids Res. 1, p. 135 et suivantes (1982)).

Les techniques utilisées, mettent en oeuvre l'utilisation concertée de ces enzymes pour couper l'ADN en des points prédéterminés, et d'enzymes dites "ligases" pour lier les fragments entre eux (P.E. Loban et A.D. Kaiser, J. Mol. Biol. 78, p. 453 et suivantes (1973)). L'ensemble est porté par des "vecteurs" (plasmides ou bactériophages), susceptibles d'être introduits dans une bactérie telle que E. coli selon des procédés connus par ailleurs, et de s'y maintenir lors de la croissance de la bactérie-hôte (M. Mandel et A. Higa, J. Mol. Biol., 53, p. 154 et suivantes (1970)).

L'expression à faible niveau du gène de l'activateur tissulaire humain du plasminogène chez E. Coli a déjà été décrite par D. Pennica et coll., Nature, 301, p. 214 et suivantes (1983). Avec les signaux d'expression utilisés, le t-PA est produit à un niveau correspondant à 50-80 microgrammes par litre de culture et par unité de densité optique.

La présente invention concerne un procédé permettant l'expression du gène de l'activateur tissulaire humain du plasminogène chez E. Coli à un niveau environ 150 fois plus élevé que celui décrit par D. Pennica et coll.

La présente invention concerne un procédé permettant d'abord d'induire la biosynthèse de l'activateur tissulaire humain du plasminogène dans un microorganisme et de convertir ensuite le t-PA produit sous une forme active.

L'invention consiste à faire d'abord une construction qui place le gène de structure du t-PA humain à côté d'un codon d'initiation, puis à lier le gène de structure ainsi modifié à un promoteur inductible.

Une bactérie-hôte, telle que E. coli, contenant le gène modifié, produit du t-PA à un niveau élevé (environ 5 % des protéines cellulaires) après induction dans des conditions définies.

La présente invention comporte également un procédé permettant de modifier la conformation du t-PA par dénaturation et renaturation afin qu'il possède une activité fibrinolytique.

Dans ce qui suit la signification des termes techniques employés en Biologie Moléculaire sera supposée connue (cf. par exemple "Biologie Moléculaire de Gène", de J. Watson, édition française Interéditions 1978). Dans ce qui suit seront décrits successivement la construction et les procédés d'expression du gène du t-PA et les procédés de dénaturation et renaturation sous une forme active du t-PA ainsi produit.

A. CLONAGE DU GENE DE L'ACTIVATEUR TISSULAIRE HUMAIN DU PLASMINOGENE

La lignée cellulaire de mélanome "Bowes" [D.C. Rijken et D. Collen, J. Biol. Chem., 256, p. 7035 et suivantes (1981)] a été utilisée pour la préparation de l'ARN messager spécifique de l'activateur tissulaire humain du plasminogène.

A partir de cellules Bowes, cultivées en monocouche en milieu Eagle modifié par Dulbecco, en présence de 10 % de sérum foetal de veau, on prépare l'ARN messager en utilisant la méthode "urée-LiCl" [C. Auffray et coll., Nucl. Acids Res., 8, p. 231 et suivantes (1980)]. On enrichit la préparation en ARN messager par plusieurs cycles de chromatographie d'affinité sur des colonnes d'oligo (dT)- cellulose, selon la technique décrite par H. Aviv et P. Leder, [Proc. Natl. Acad. Sci (USA), 69, p. 1408 et suivantes (1972)].

La préparation de l'ARN messager, spécifique pour l'activateur tissulaire humain du plasminogène, a été contrôlée en utilisant la technique d'hybridation "Northern" [P.S. Thomas, Proc. Natl. Acad. Sci. (USA), 77, p. 5201 et suivantes (1980)]. A cet effet, on utilise un oligonucléotide synthétique codant pour les acides aminés 11-17 selon la séquence publiée par D. Pennica et coll., Nature, 301, p. 214 et suivantes (1983). Après mesure de la radioactivité des bandes, on détermine, par le calcul, que la concentration de l'ARN spécifique pour l'activateur tissulaire humain du plasminogène représente 0,03 % de l'ARN total.

L'ARN messager ainsi préparé est utilisé pour synthétiser in vitro l'ADN complémentaire (ADNc) en utilisant les techniques décrites par A. Efstratiadis et coll., J. Biol. Chem., 7, p. 279 et suivantes (1976) et par M.P. Wickens et coll., J. Biol. Chem., 253, p. 2483 et suivantes (1978).

Pour l'amorce de l'ADNc on utilise un oligonucléotide spécifique pour les acides aminés 511-527 de l'activateur tissulaire humain du plasminogène (d'après la séquence publiée par D. Pennica et coll., Nature, 301, p. 214 et suivantes (1983) et un oligo-(dT).

Après avoir digéré l'ADNc avec la nucléase S1 (A. Efstratiadis et coll., J. Biol. Chem., 7, p. 279 et suivantes (1976), on méthyle les sites potentiellement présents selon la technique décrite par T. Maniatis et coll., Cell., 15, p. 687 et suivantes (1978).

Après addition des adaptateurs EcoRl, l'ADNc ainsi modifié est digéré par l'enzyme de restriction EcoRl puis est ensuite inséré dans le site EcoRl du vecteur Lambda-gt10 (T.V. Huynh et coll., DNA Cloning, D.M. Glover IRL Press ed., p. 49 et suivantes (1985).

A partir de 0,6 microgrammes d'ADNc, double brin, on établit une banque d'environ 213000 clones (unités formant des plages).

La recherche d'un clone portant le gène de l'activateur tissulaire humain du plasminogène est conduite à l'aide de 3 sondes oligonucléotidiques. La séquence de ces 3 sondes (D. Pennica et coll., Nature, 301, p. 214 et suivantes (1983) correspond à la spécificité suivante :

| Sonde | Acides aminés codés |
|-------|---------------------|
| 1     | 11-18               |
| 2     | 425-432             |
| 3     | 511-527             |

Par hybridation de ces sondes aux plages transférées sur des filtres de nitrocellulose (T. Maniatis et coll., Molécular Cloning, Cold Spring Harbor Laboratory, p. 309 et suivantes (1982), on isole plusieurs clones codant potentiellement pour l'activateur tissulaire humain du plasminogène.

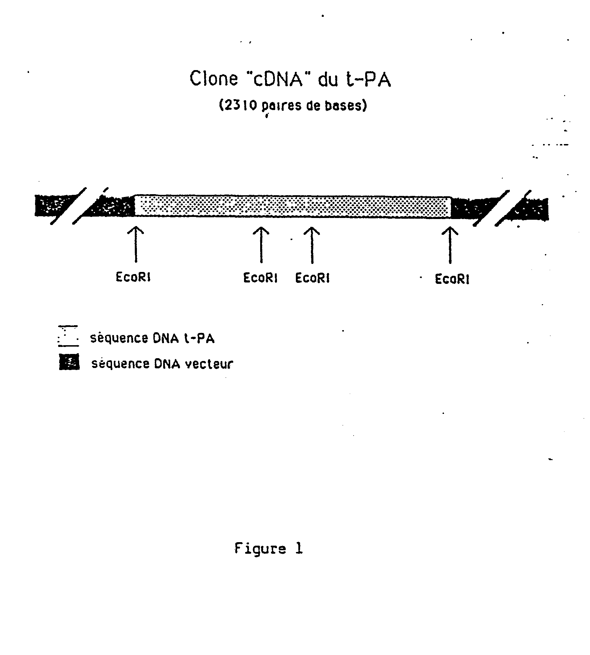

Les analyses de restriction permettent d'aboutir à un clone d'ADNc codant pour l'ensemble du gène dont la structure générale est donnée dans la figure 1.

Pour confirmer l'authenticité du clone isolé, on détermine la séquence nucléotidique du gène. Le fragment d'insertion EcoRl-EcoRl est séquencé par la technique de dégradation chimique partielle décrite par A. Maxam et W. Gilbert, [Proc. Natl. Acad. Sci. (USA), 74, p. 560 et suivantes (1977)] et par la technique "dideoxy" décrite par F. Sanger et A.R. Coulson, J. Mol. Biol., 94, p. 441 et suivantes (1975).

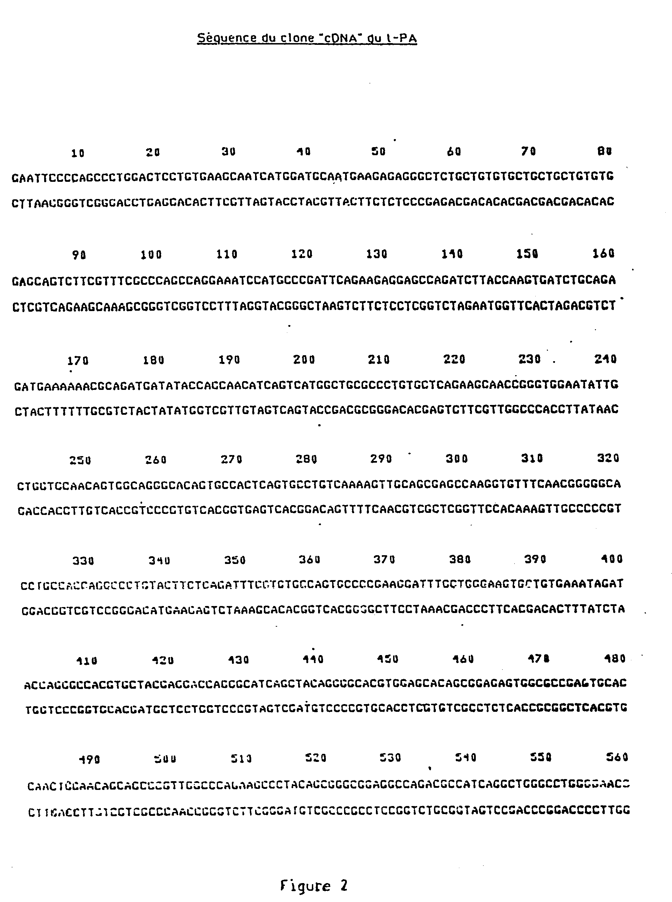

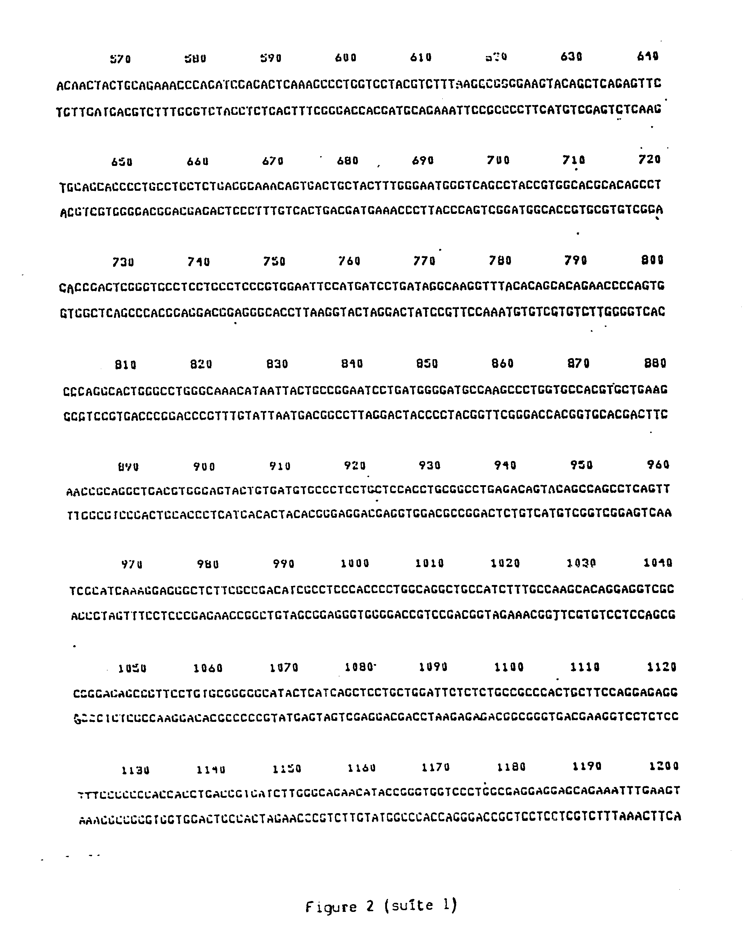

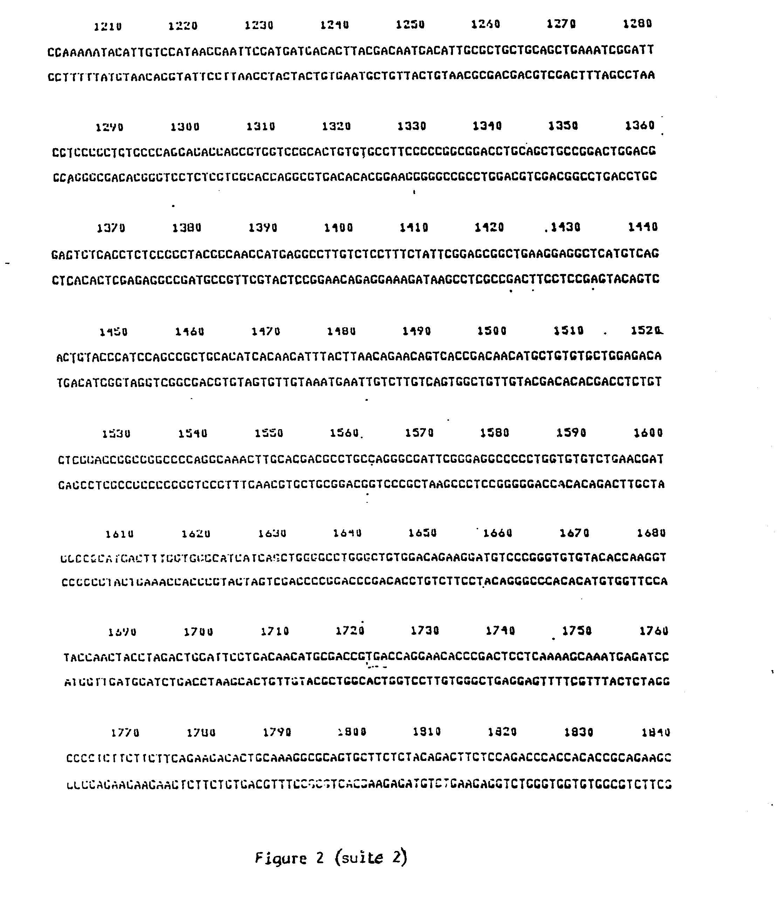

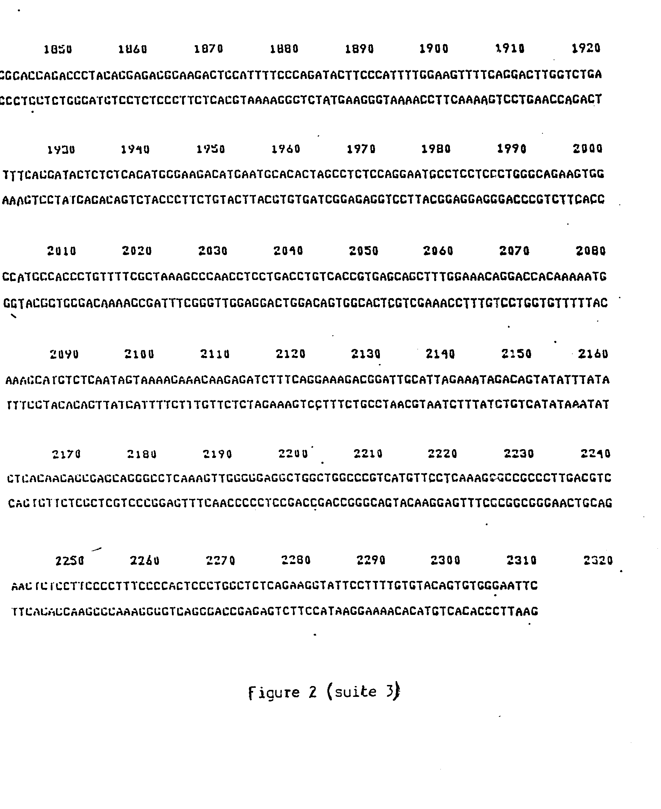

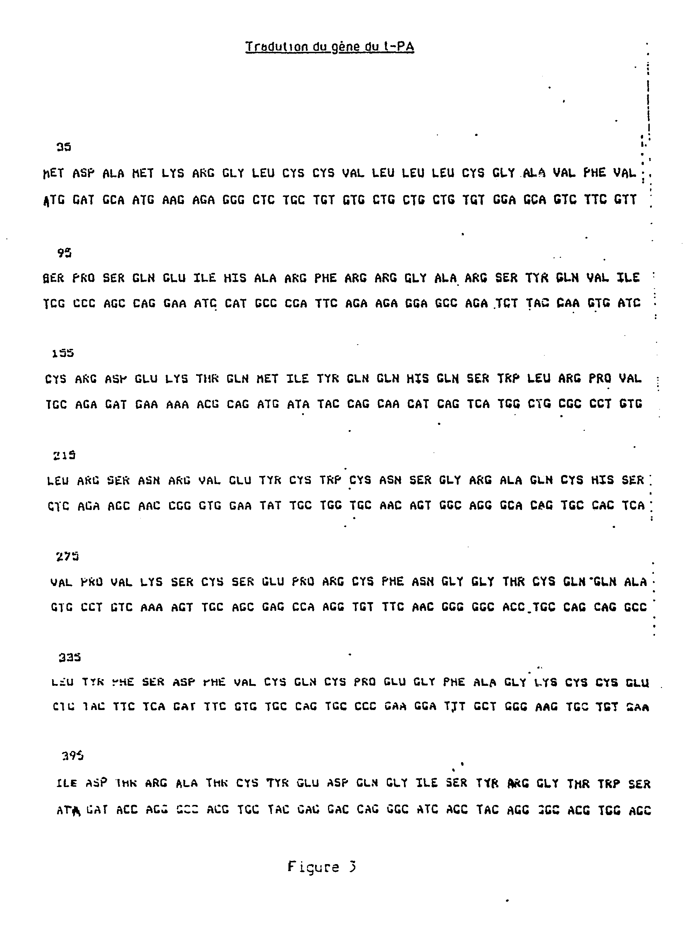

La séquence obtenue à partir de ces deux méthodes est indiquée dans la figure 2. La partie codante ne présente aucune différence avec la séquence publiée par D. Pennica et coll. Cependant des différences sont constatées dans les régions non-codantes, mais elles ne concernent pas l'expression hétérologue du gène de l'activateur tissulaire humain du plasminogène chez E. Coli.

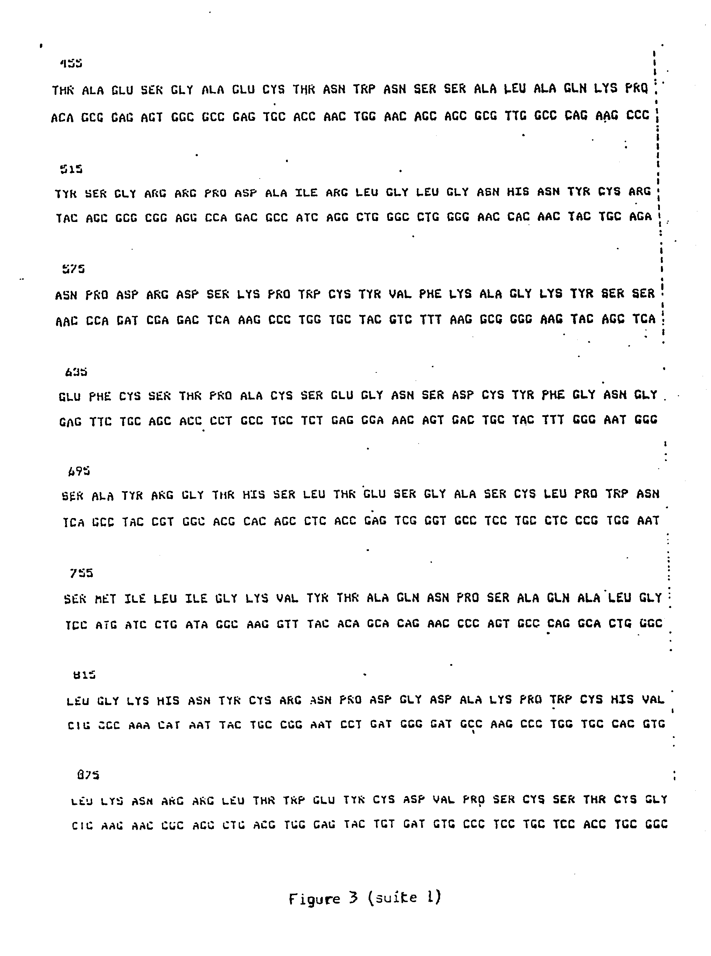

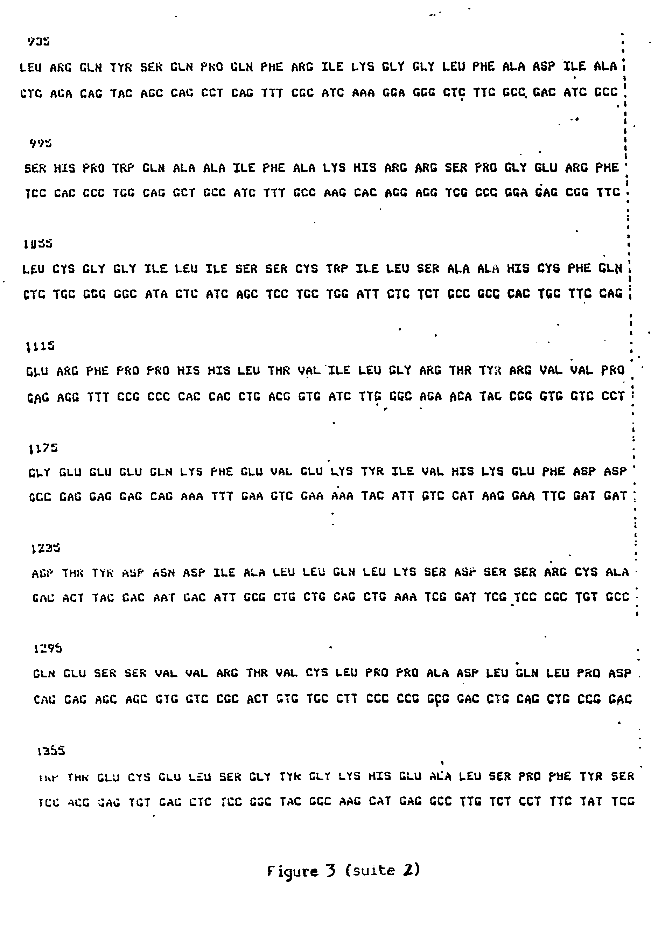

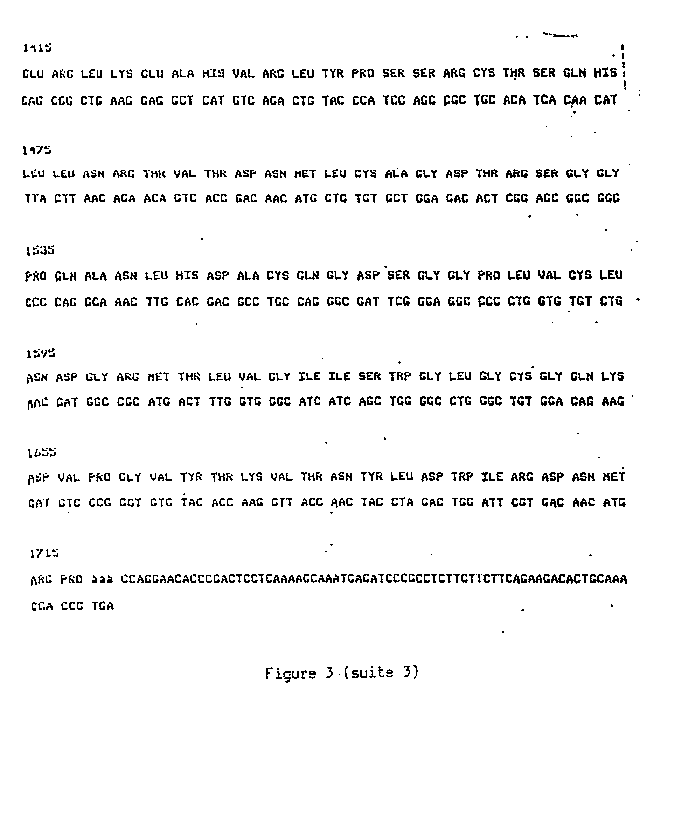

Le produit de traduction, selon D. Pennica et coll., Nature, <u>301</u>, p. 214 et suivantes (1983), est représenté sur la figure 3.

<u>B. CONSTRUCTION DU PLASMIDES RECOMBINANTS EN VUE DE L'EXPRESSION DU t-PA SOUS LE CONTROLE DU PROMOTEUR $P_L$ DU BACTERIOPHAGE LAMBDA</u>

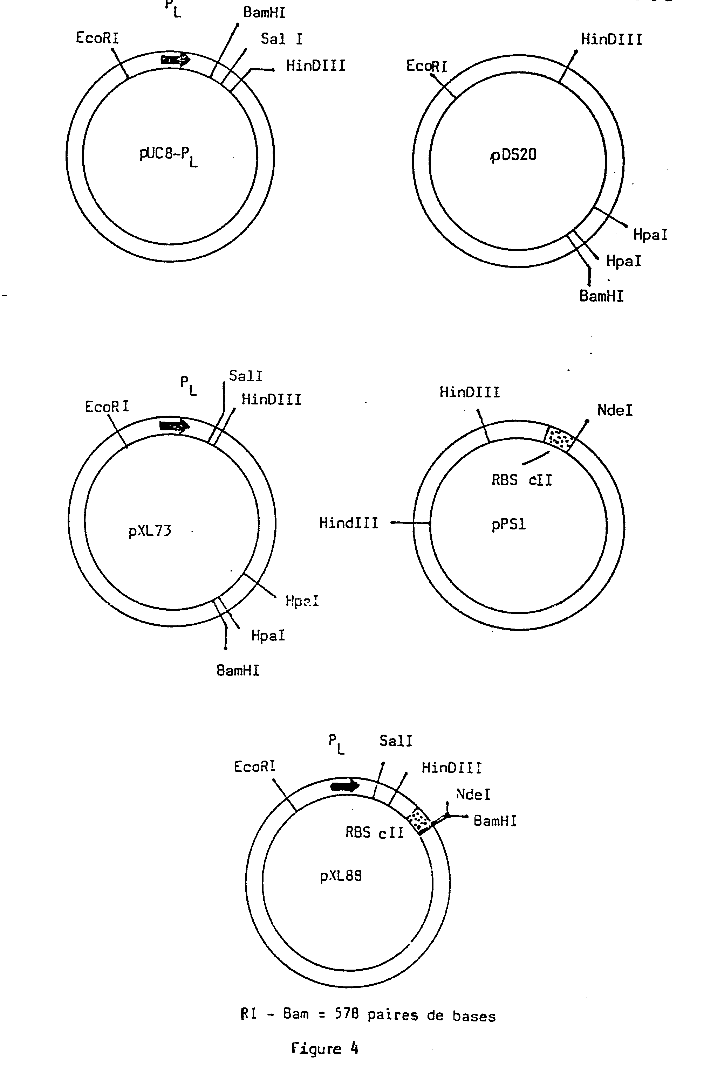

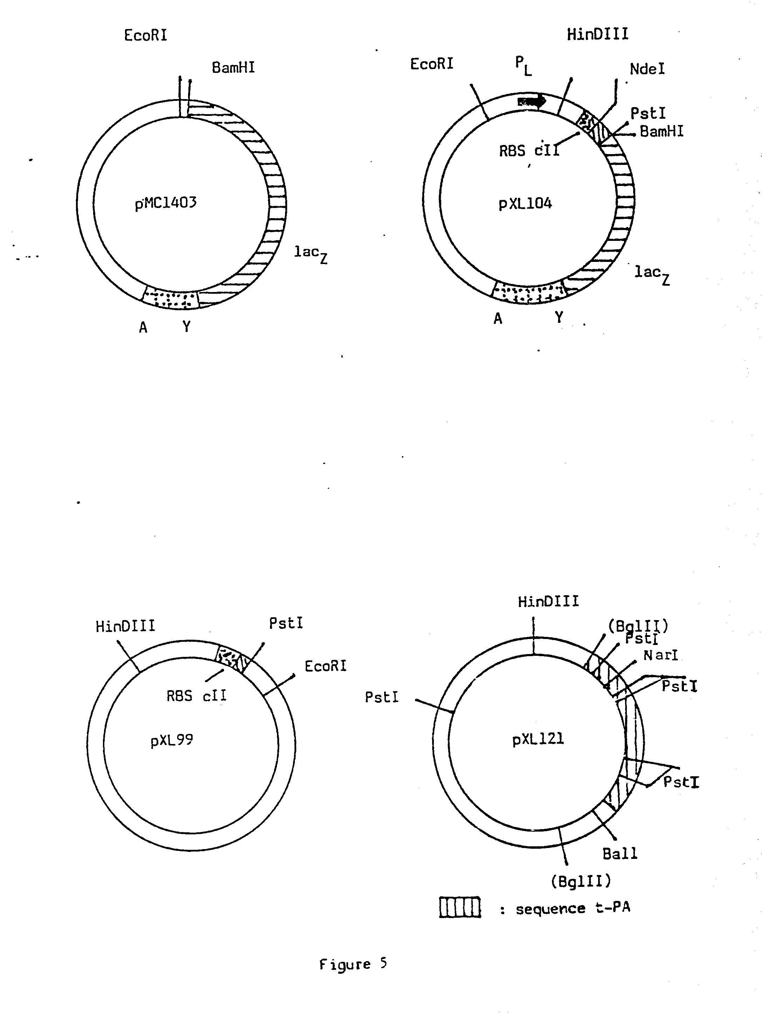

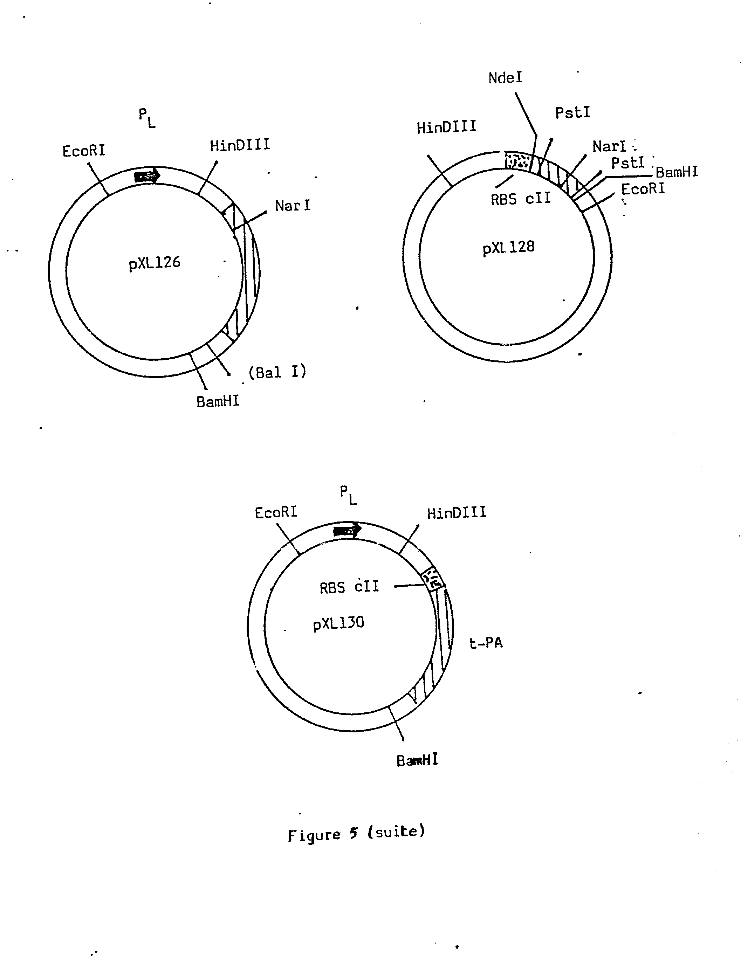

1) <u>Isolement des signaux d'expression</u> (Fig.4)

les signaux d'expression décrits dans la présente invention sont :

- le promoteur gauche $P_L$ du bactériophage lambda qui permet une transcription efficace de l'ADN en ARN messager,

- le site de fixation des ribosomes du gène cII du bactériophage lambda qui permet une traduction efficace du ARN messager.

Le promoteur "$P_L$" du bactériophage lambda est placé sur le chromosome du bactériophage entre un site BglII et un site BamHI (voir E. Szybalski et W. Szybalski, Gene <u>7</u>, p. 217 et suivantes (1979)), dont la séquence nucléotidique est connue (F. Sanger et coll., J. Mol. Biol, <u>162</u>, p. 279 et suivantes (1982)). On peut cloner ce fragment et modifier ses sites de restriction selon des méthodes connues.

On note que les plasmides portant $P_L$ doivent être propagés dans des souches de <u>E. coli</u> portant le gène répresseur cI, afin d'éviter que ce promoteur ne s'exprime de façon constitutive.

Dans une première construction, $P_L$ est disponible sous forme d'un fragment BamHI à partir du plasmide "$pP_L$-lambda" (Pharmacia P.L. Biochemicals). A partir de ce plasmide, on peut isoler un fragment HaeIII-HaeIII contenant $P_L$ et insérer ce fragment dans le site SmaI de "pUC8", (J. Veira et J. Messing, Gene 79, p. 250 et suivantes (1982)) pour obtenir "pUC8-$P_L$".

La séquence du promoteur étant alors disposée de part et d'autre de nombreux sites de restriction, le fragment qui porte ce promoteur peut être excisé et combiné de manière adéquate à d'autres fragments d'ADN.

Par exemple, on peut après avoir détruit le site BamHI de "pUC8-$P_L$" par action de l'enzyme S1 (Boehringer) isoler un fragment EcoRI-HinDIII contenant le promoteur $P_L$ et ensuite lier ce fragment avec le grand fragment EcoRI-HinDIII du plasmide "pDS20" (G. Duester et coll., Cell 30, p. 855 et suivantes (1982) pour obtenir le plasmide "pXL73".

Le gène cII du bactériophage lambda, dont la séquence et le site d'initiation sont connus, peut être traduit avec efficacité [E. Schwarz et coll. Nature 272, p. 410 et suivantes (1978)]. En utilisant le RBS ("ribosome binding site" ou "site de fixation des ribosomes") de ce gène, on construit un plasmide contenant le système d'expression "Promoteur $P_L$-RBS cII-ATG-site de restriction". Le RBS du gène cII est extrait du plasmide "pPS1" (P. Sarmientos et coll., Cell, 32, p. 1337 et suivantes (1983). On digère ce plasmide par NdeI et on insère un adaptateur BamHI après formation d'extrémités franches. On excise alors le RBS sous forme d'un fragment HinDIII-BamHI.

On construit alors un plasmide "pXL88" dans lequel ce fragment HinDIII-BamHI est lié avec le grand fragment HinD III-BamHI du plasmide "pXL73". Dans le nouveau plasmide "pXL88" le RBS cII est inséré dans la bonne orientation par rapport au promoteur $P_L$, le tout dans un système multisites de telle sorte que l'ensemble $P_L$-RBS cII soit porté sur un seul fragment d'ADN.

2) <u>Fusion du gène "cDNA" du t-PA aux signaux d'expression</u>
   (Fig. 5)

Après avoir séquencé la partie codante du gène du t-PA, on prépare deux oligonucléotides synthétiques complémentaires codant pour les 7 premiers acides aminés du t-PA mature (Fig. 3, de position 140 à position 160). Ce petit fragment d'ADN synthétique peut être fabriqué de telle sorte qu'il soit clonable comme un fragment NdeI-BamHI, par exemple :

    5' TATGTCTTACCAAGTGATCTGCAGA       3'
    3'    ACAGAATGGTTCACTAGACGTCTCTAG  5'

On réalise la ligation de ce fragment NdeI-BamHI avec les fragments d'ADN suivants :

a) un fragment EcoRI-NdeI du plasmide "pXL88" portant le promoteur $P_L$ et le site d'initiation de la traduction (RBS) du gène cII.

b) un fragment EcoRI-BamHI du plasmide "pMCl403" (M.J. Casadaban et coll., J. Bacteriol. <u>143</u>, p. 971 et suivantes (1980)) portant un gène de résistance aux antibiotiques, l'origine de réplication et l'extrémité 3' du gène de la béta-galactosidase.

On isole un plasmide, "pXL104", qui correspond à une fusion des 7 premiers acides aminés du t-PA mature avec la béta-galactosidase d'<u>E.coli</u>. Le plasmide "pXL104" porte en amont une fusion du RBS cII avec les codons correspondant aux 7 premiers acides aminés du t-PA mature.

La présence d'un site PstI à l'emplacement du sixième acide aminé du t-PA mature permet l'excision d'un fragment HinDIII-PstI contenant le RBS cII suivi par le début du t-PA mature. On insère ce fragment entre les sites HinDIII et PstI du plasmide "pUC9" (J. Veira et J. Messing, supra) pour obtenir le plasmide "pXL99".

Le gène "ADNc" du t-PA ayant été cloné dans le génome du bactériophage lambda (Fig. 1), on digère l'ADN de ce bactériophage recombinant par l'enzyme BglII pour isoler un fragment de 1974 paires de bases contenant la séquence codant pour le t-PA mature.

Tirant profit de la compatibilité entre les sites BglII et BamHI, on insère ce fragment BglII-BglII dans le site BamHI du plasmide "pBR322" (Pharmacia, P.L. Biochemicals) pour obtenir le plasmide "pXL121".

L'insertion du gène "ADNc" du t-PA dans un plasmide permet l'utilisation d'autres sites de restriction qui sont très fréquents dans le génome du bactériophage lambda.

A partir du plasmide "pXL121" on peut, par exemple, construire d'autres plasmides dans lesquels des fragments du gène, ou le gène complet, sont fusionnés aux signaux d'expression.

Dans une première construction, on digère l'ADN du plasmide "pXL121" par les enzymes HinDIII et BalI et on obtient un fragment d'ADN contenant la totalité de la séquence codante. On effectue une ligation du fragment HinDIII-BalI ainsi obtenu avec le grand fragment HinDIII-HpaI de "pXL73". Cette ligation est possible car les enzymes BalI et HpaI donnent, après coupure, des extrémités franches. On aboutit alors au plasmide "pXL126" dans lequel le gène du t-PA est fusionné au promoteur $P_L$ mais le plasmide "pXL126" ne contient pas de RBS.

Le plasmide "pXL99", décrit ci-dessus contient le RBS cII lié au codons correspondant aux 6 premiers acides aminés du t-PA. La présence d'un site PstI au sixième codon permet l'accrochage à la suite de la séquence du t-PA. Plusieurs sites étant contenus dans le gène du t-PA il n'est pas possible de réaliser une construction simple.

On peut isoler un fragment PstI-PstI de 414 paires de bases codant pour les acides aminés 7-144 du t-PA, après coupure de plasmide "pXL121" par l'enzyme PstI. On insère ce fragment d'ADN dans le site PstI du plasmide "pXL99" et, après avoir vérifié l'orientation de ce fragment, on obtient un nouveau plasmide "pXL128" dans lequel de RBS cII est maintenant lié à la séquence codant pour les 144 premiers acides aminés du t-PA.

Au 110ème codon du gène de t-PA un site NarI est présent. Par conséquent, on peut exciser hors du plasmide "pXL128" un fragment HinDIII-NarI contenant le RBS cII et l'extrémité 5' du gène du t-PA. On insère ce fragment dans "pXL126" à la place d'un fragment équivalent HinDIII-NarI mais qui ne porte pas le RBS cII.

Cette construction conduit au plasmide "pXL130" qui contient le promoteur $P_L$, le site de fixation des ribosomes RBS cII, un codon d'initiation ATG et le gène de structure du t-PA et qui permet d'exprimer le t-PA dans E. Coli.

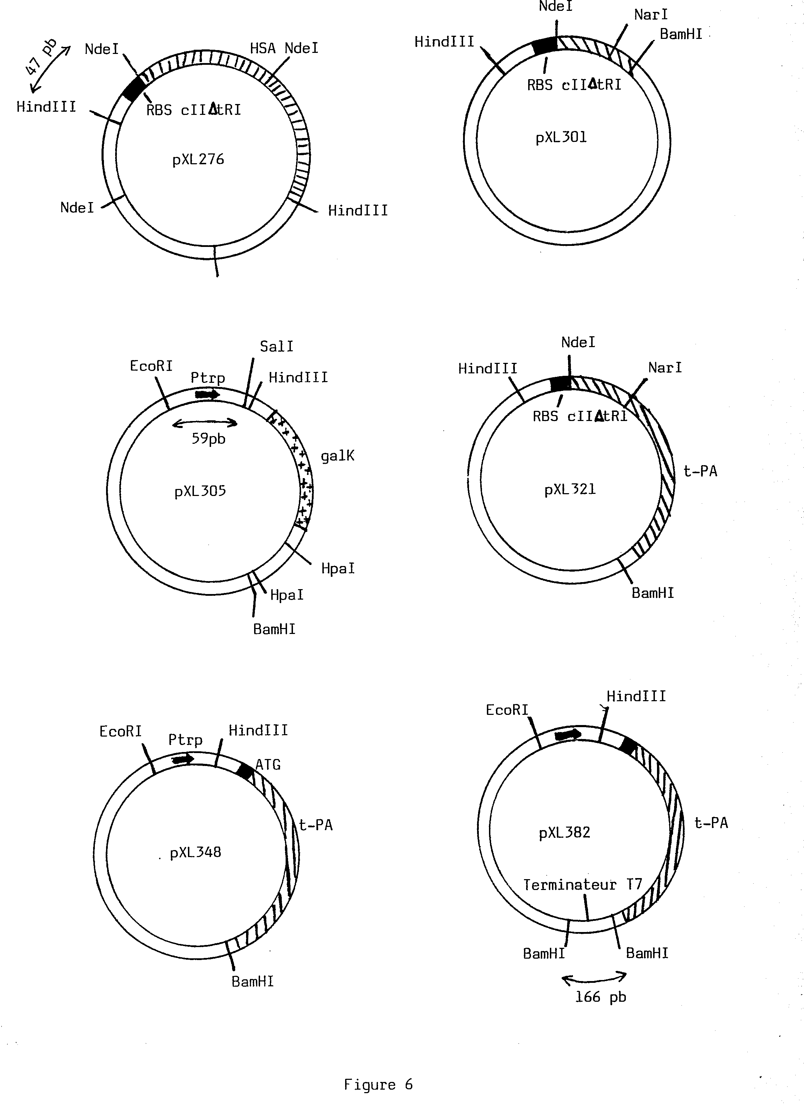

C. CONSTRUCTION DE PLASMIDES RECOMBINANTS EN VUE DE L'EXPRESSION DU t-PA SOUS LE CONTROLE DU PROMOTEUR Ptrp DE L'OPERON TRYPTOPHANE DE E.COLI (Figure 6)

Il est également possible, en procédant comme suit, d'obtenir des constructions permettant d'exprimer le t-PA dans E.coli sous le contrôle du promoteur de l'opéron tryptophane de E.coli (Ptrp).

On peut isoler un fragment HinDIII-NdeI de 47 paires de bases contenant le RBS cII délété du terminateur tR1 à partir d'une digestion par HinDIII et NdeI du plasmide pXL276. Ce plasmide est décrit dans la demande de brevet européen EP 198745. Ce fragment peut être combiné au fragment NdeI-BamHI du plasmide pXL128 contenant la séquence codant pour les 144 premiers résidus du tPA, le tout étant inséré entre les sites HinDIII et BamHI du plasmide pUC8 (J. Veira et J. Messing, cf. supra) pour donner le plasmide pXL301.

On construit alors le plasmide pXL321 dans lequel le fragment HinDIII-NarI de pXL301 contenant le RBS cII délété du terminateur tR1 (RBS cII∆tR1) et l'extrémité 5' du gène du t-PA est inséré entre les sites HinDIII et NarI du plasmide pXL126 à la place d'un fragment équivalent HinDIII-NarI mais qui ne porte pas le RBS cII. On reconstitue ainsi le gène complet du t-PA.

Par ailleurs, on construit le plasmide pXL305 en remplaçant le fragment EcoRI-SalI de pXL73 contenant le promoteur $P_L$ par un fragment EcoRI-SalI de 59 paires de base provenant du plasmide pDR720 (Pharmacia) et contenant le promoteur Ptrp.

Le gène du t-PA peut alors être placé sous le contrôle du promoteur Ptrp en insérant le fragment HinDIII-BamHI de pXL305, en remplacement d'un fragment HinDIII-BamHI contenant le gène galK de la galactokinase d'E.coli. On obtient ainsi le plasmide pXL348.

Il a de plus été observé que l'insertion en aval du gène structural du t-PA d'un signal efficace de terminaison de la transcription était susceptible d'avoir un effet bénéfique sur l'expression du t-PA à partir du promoteur tryptophane.

On peut, par exemple, utiliser le site de terminaison de la transcription de la fin de la région précoce du bactériophage T7 ("terminateur T7") (J.J. Dunn et F.W. Studier, Nucléic Acids Res. $\underline{8}$, P.2119 et suivantes, [1980]). Le terminateur T7 est disponible sur un fragment BamHI de 166 paires de bases du plasmide pAR-4 (Don du Dr Imre Boros, Institute of Biochemistry, Biological Research Center, P.O.B. 521 H-6701 SZEGED, Hongrie).

On obtient finalement le plasmide pXL382 en insérant, dans la bonne orientation, le fragment BamHI de pAR-4 décrit ci-dessus dans le site unique BamHI de pXL348. Le plasmide pXL382 porte donc la séquence "Ptrp-RBS cII $\Delta$ tRl-codon ATG gène de structure du tPA-Terminateur T7".

## D. EXPRESSION DU GENE t-PA HUMAIN CHEZ E. COLI

Dans le plasmide "pXL130" la transcription du gène du t-PA est dirigée par la promoteur $P_L$. Pour éviter la transcription néfaste due à ce promoteur lors de la propagation du plasmide, il est nécessaire d'utiliser des bactéries hôtes qui codent pour le répresseur cI du bactériophage lambda. De cette manière une souche contenant le plasmide "pXL130" peut être cultivée en conditions de répression du promoteur $P_L$. Sans cette précaution, l'expression constitutive de t-PA rend les cellules malades, incapables de se reproduire et le taux de production de t-PA est peu élevé dans la culture.

On peut utiliser, comme système d'induction conditionnel pour le plasmide "pXL130", une souche d'E.coli contenant un gène répresseur thermosensible (cI-ts). Lorsque la bactérie est en phase exponentielle, on induit le promoteur $P_L$ du plasmide en élevant très rapidement la température d'incubation à 42°C.

Dans ces conditions, le t-PA est synthétisé à 42°C et cette température doit être maintenue pour garder $P_L$ actif.

Dans une souche d'E. coli telle que N4830 (Pharmacia, P.L. Biochemicals), on effectue l'incubation pendant 90 minutes. On prélève un échantillon de la culture et on lyse les bactéries par sonication. Le t-PA, présent dans la fraction insoluble, représente environ 1 % des protéines totales fabriquées par E. coli.

Il a maintenant été trouvé, et c'est ce qui constitue un autre objet de la présente invention, que la production de l'activateur tissulaire humain du plasminogène peut être considérablement améliorée en utilisant une souche codant pour la protéine "cro" du bactériophage lambda.

En particulier, il s'agit d'une souche cI-ts où le gène "cro" est exprimé après l'inactivation thermique du répresseur cI. Par exemple, dans le cas d'une souche comme OR1319 (O. Reyes, Virology 123, p. 357 et suivantes (1982)), on fait pousser les bactéries à 30°C, température à laquelle ni t-PA, ni "cro", ne sont exprimés. Lorsque les bactéries sont en phase exponentielle, on élève très rapidement la température d'incubation à 42°C. Le répresseur cI est maintenant inactivé et le gène "cro" peut être exprimé. Après 30 minutes d'incubation à 42°C, on fait revenir la température à 30°C. La présence de la protéine "cro" empêche la synthèse-de novo du répresseur cI et maintient $P_L$ actif (G.N. Gussin et coll., dans R.W. Hendrix, J.W. Roberts, F.W. Stahl et R.A. Weisberg (eds), Lambda II, Cold Spring Harbour Laboratory Press, p. 93 et suivantes (1983)). On continue l'incubation pendant 90 minutes. Dans ces conditions, le t-PA produit représente environ 5 % des protéines totales de E. coli et il peut être récupéré avec un rendement d'environ 10 mg par litre de culture et par unité de densité optique.

Le plasmide pXL382 contient le gène fonctionnel du tPA sous le contrôle du promoteur de l'opéron tryptophane d'E.coli (Ptrp).

L'expression du tPA est donc induite dans ce cas lorsque la bactérie est cultivée en absence de tryptophane. Il est de plus indispensable d'utiliser comme hôte du plasmide pXL382 une souche E.coli trpR$^+$, c'est-à-dire exprimant le répresseur de l'opéron tryptophane à un niveau au moins égal à celui d'une souche sauvage. (B.P. Nichols et C. Yanofsky, Methods Enzymol. (1983), 101, P.155 et suivantes). On peut utiliser, par exemple, la souche E.coli B 54125 (collection de l'Institut Pasteur). On procède, par exemple, de la façon suivante : à partir d'un réisolement récent de la souche E.coli B (pXL382) sur une boîte de Pétri gélosée à base de milieu LB (J.H. Miller, "Experiments in Molecular Biology", Cold Spring Harbor Laboratory, New York, [1972], P.431 et suivantes) contenant 50 μg/cm$^3$ d'ampicilline, on prépare une préculture en milieu synthétique contenant 100 μg/cm$^3$ de tryptophane (par exemple, milieu M9 glucosé contenant 0,4 % casamino acids selon J.H. Miller, cf. supra) à 37°C avec agitation pendant 16 heures. Cette préculture est ensuite utilisée pour ensemencer au 1/100ème un milieu identique mais ne contenant que 2 μg/cm$^3$ de tryptophane. La culture de production est réalisée à 37°C avec agitation. Dans des conditions de laboratoire (culture en erlenmeyer), on récolte les cellules vers la fin de la phase exponentielle, après environ 6 heures de culture. La turbidité est alors comprise entre 2 et 3 unité de densité optique à 610 nm.

Les rendements obtenus sont comparables à ceux décrits ci-dessus, soit de l'ordre de 5 à 10 mg de tPA par litre de culture et par unité de densité optique à 610 nm, ce qui représente environ 5 % des protéines totales de E.coli.

E. RENATURATION DU t-PA FABRIQUE DANS E. COLI

Les protéines hétérologues synthétisées à haut niveau chez E. coli se retrouvent souvent dans la cellule sous forme d'aggrégats insolubles (R.G. Schoner et coll., Biotechnology 3, p.151 et suivantes (1985)). Il a été trouvé que c'est le cas pour le t-PA exprimé à haut niveau. Avec ce procédé de production, la grande majorité du t-PA se retrouve dans le culot après centrifugation à basse vitesse alors que le t-PA est normalement soluble en solution aqueuse. Une explication possible de l'insolubilité du t-PA produit dans E. coli est que la protéine ne se trouve pas synthétisée dans la forme native. Il a été en effet montré dans d'autres systèmes que des protéines mal repliées ont tendance à s'aggréger et à précipiter (J. London et coll., Eur. J. Biochem. 47, p. 409 et suivantes (1974) ; G. Orsini et M.E. Goldberg, J. Biol. Chem. 252, p. 3453 et suivantes (1978)). Dans sa forme non native une protéine enzymatique n'est pas active et l'on peut donc raisonablement prévoir que le t-PA synthétisé chez E. coli n'est pas sous sa forme active.

De nombreuses protéines peuvent revenir à leur forme native in vitro après dénaturation et réduction contrôlée. Cela a été vérifié notamment pour la tryptophanase d'E. coli, le chymotrypsinogène, l'anhydrase carbonique, la ribonucléase, le lysozyme et l'inhibiteur trypsique de pancréas bovin (J. London et coll., supra ; G. Orsini et M.E. Goldberg, supra ; U. Carisson et coll., Eur. J. Biochem, 52, p. 25 et suivantes (1975) ; R.F. Goldberger et coll., J. Biol. Chem. 239, p.1406 et suivantes (1964) ; V.P. Saxena et D.B. Wetlaufer, Biochemistry 9, p. 5015 et suivantes (1970) ; T.E. Creighton, J. Mol. Biol. 87, p. 563 et suivantes (1974)). Dans la majorité des cas, l'indicateur le plus sensible de renaturation complète est l'activité enzymatique. Lorsqu'une protéine donnée porte deux activités, les deux activités sont retrouvées à la fois ce qui semble exclure la possibilité qu'une fraction de la population de molécules puisse exister avec seulement une partie de structure correcte.

Le t-PA humain a deux propriétés facilement mesurables : la capacité d'activer le plasminogène à un certain niveau et l'affinité pour la fibrine manifestée par l'activation du t-PA lui-même.

Il a été trouvé, et c'est ce qui fait aussi l'objet de la présente invention, que le t-PA produit par E. Coli, peut être renaturé in vitro sous une conformation active après avoir complètement dénaturé et réduit la chaîne polypeptidique.

Après la lyse des bactéries par sonication dans une solution isotonique telle que, par exemple, du P.B.S. (0,2 g/l KCl ; 0,2 g/l $KH_2PO_4$ ; 8 g/l NaCl et 1,25 g/l $Na_2HPO_4$), le t-PA se trouve dans la fraction insoluble. Il est donc possible d'obtenir à ce niveau une purification importante de cette enzyme en centrifugeant le lysat cellulaire et en récupérant uniquement le culot de centrifugation. Le t-PA représente alors 20-25 % des protéines insolubles présentes dans le culot.

Il a été trouvé qu'un réarrangement des structures secondaire et tertiaire accompagné d'une réduction et d'une réoxydation de la chaîne polypeptidique est nécessaire pour obtenir la forme active et soluble du t-PA.

Le t-PA est d'abord solubilisé en conditions dénaturantes et réductrices en traitant la fraction insoluble avec une solution :

7 M guanidine HCl

0,1 M béta-mercaptoethanol

0,1 M $KH_2PO_4$ pH7,5

(voir paragraphe F ci-dessous).

Pour permettre ensuite à la molécule de se renaturer il est nécessaire d'éliminer ou de diminuer les concentrations des agents dénaturants et réducteurs. On peut, par exemple, éliminer les effets de ces agents par dilution de la fraction dénaturée/réduite dans des tampons permettant la renaturation. Plus particulièrement, la renaturation du t-PA peut être obtenue par dilution dans la solution suivante qui est un exemple obtenu après une série d'optimisations :

2,5 M urée

50 mM Tris HCl pH 8,75

10 mM NaCl

5 mM EDTA

1 mM glutathion réduit

0,3 mM glutathion oxydé

10 mM lysine

et selon la méthode décrite dans le paragraphe F ci-dessous.

On a quantifié l'efficacité de la renaturation en mesurant l'activité du t-PA sur des plaques de fibrine-agarose selon la méthode décrite par A. Granelli-Piperno et E. Reich, J. Exp. Med. 148, p.223 et suivantes (1978). Après avoir comparé cette activité à celle obtenue avec un t-PA simple-chaine standard (Biopool, Suède), on peut mesurer un rendement d'environ 3 mg de t-PA actif par litre et par unité de densité optique de la culture bactérienne.

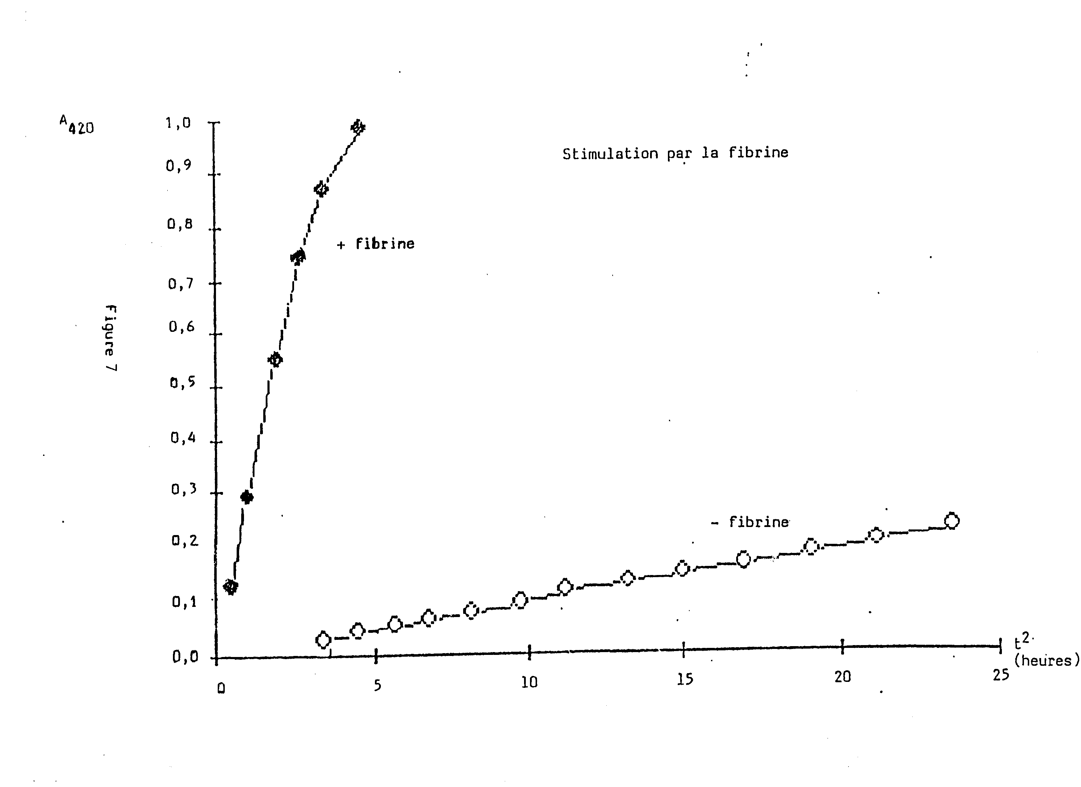

L'activité du t-PA peut être aussi mesurée par l'hydrolyse d'un substrat synthétique (J.H. Verheijet et coll., Thromb Haemostas (Stuttgart) 48, p. 256 et suivantes (1982)). En particulier, on mesure la vitesse de conversion du plasminogène en plasmine par le t-PA. Des incubations en présence de plasminogène humain purifié et d'un substrat synthétique de la plasmine (S-2251 : Val-leu-Lys-Paranitroaniline, Kabi Vitrum, Suède) permettent de mettre en évidence des cinétiques de type exponentiel caractéristiques de l'activateur du plasminogène (fig.7). Des incubations témoins en absence de plasminogène permettent de constater que les extraits ne contiennent pas d'activités protéolytiques pouvant donner de fausses réponses positives.

Les extraits protéiques obtenus à partir de bactéries ne contenant pas le gène de l'activateur du plasminogène et ayant subi le même traitement de renaturation se sont révélés totalement inactifs après incubation en présence de plasminogène et de substrat synthétique S-2251. Seuls les extraits obtenus à partir de bactéries porteuses du gène de t-PA manifestent une activité enzymatique du type activateur du plasminogène.

La même technique permet de tester le rôle potentiel de la fibrine sur la catalyse du plasminogène en plasmine. L'activateur tissulaire du plasminogène synthétisé par les cellules mammifères est fortement stimulé par la fibrine ou ses dérivés. Cette propriété est responsable de son intérêt thérapeutique. En effet, en absence de fibrine, l'affinité de l'enzyme pour son substrat est faible. Au contraire, en présence de fibrine, l'affinité de l'enzyme pour son substrat est augmentée. Il en résulte un enzyme pleinement actif qui coupe le plasminogène en plasmine, cette dernière dégradant le caillot de fibrine.

Cette propriété de stimulation de la fibrine sur les molécules obtenues par recombinaison génétique et renaturation peut être mise en évidence en déterminant des cinétiques enzymatiques en présence du plasminogène humain purifié, de substrat synthétique S-2251 et de fibrine ou de ses dérivés (fibrinopeptides). On observe une stimulation très importante de la vitesse de catalyse qui se trouve multipliée par un facteur d'environ 40 (fig. 7).

Par ailleurs, le t-PA recombinant, issu de E. Coli, et renaturé selon le procédé de la présente invention, peut être reconnu par des anticorps spécifiques de l'activateur du plasminogène synthétisé par les cellules mammifères.

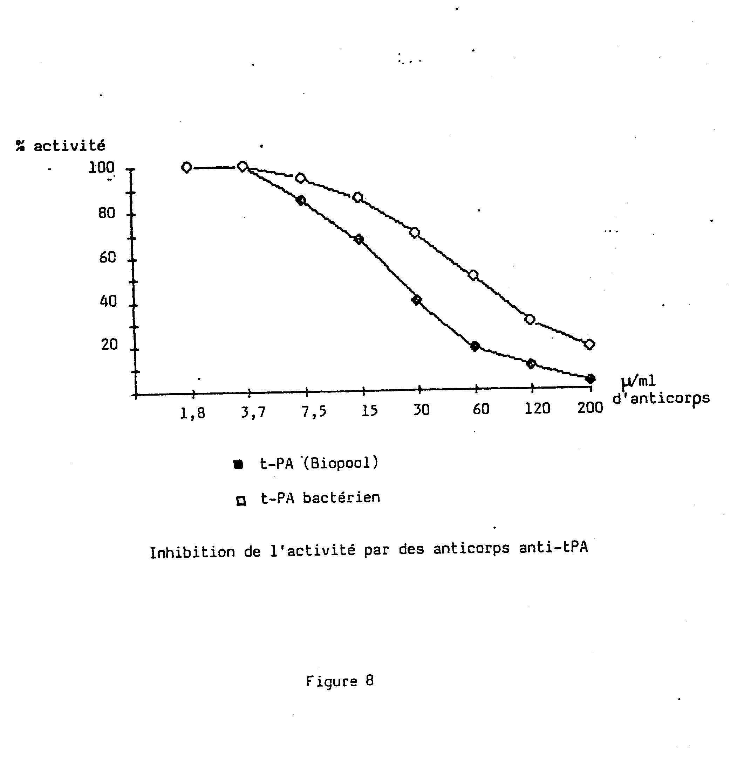

A cet effet, des quantités croissantes d'immunoglobulines purifiées spécifiques de l'activateur tissulaire humain du plasminogène (Biopool, Suède) sont incubées en présence de t-PA bactérien et l'effet sur l'activité enzymatique est analysé. Les résultats donnés dans la figure 8 montrent que l'activité du t-PA bactérien est inhibée par les anticorps spécifiques de l'activateur humain.

Cette inhibition est spécifique puisque l'activateur du plasminogène d'origine bactérienne, comme l'activateur du plasminogène d'origine humaine, n'est inhibé ni par des immunoglobulines d'animal non immunisé, ni par des immunoglobulines spécifiques de l'urokinase (Biopool, Suède).

L'ensemble de ces résultats montre que les bactéries porteuses du gène de l'activateur tissulaire humain du plasminogène synthétisent la protéine correspondante. Cette protéine n'est pas soluble dans les tampons conventionnels et n'est probablement pas dans sa conformation native. Cependant, après extraction, dénaturation et renaturation ménagée, il est possible de redonner à la protéine les sites stéréochimiques responsables de l'activité catalytique vis-à-vis du plasminogène et de l'affinité pour la fibrine. De plus, la molécule renaturée est reconnue par les anticorps anti-activateur humain. Il en résulte que la structure secondaire et tertiaire de la protéine obtenue est voisine de celle de la protéine naturelle.

## F. DESCRIPTION DES METHODES EMPLOYEES

Les enzymes de restriction sont employées selon les suggestions du fournisseur (New England Biolabs). Les ligations des fragment d'ADN sont réalisées dans des tampons contenant 50 mM Tris HCl pH 7,4 10 mM MgCl$_2$, 1 mM ATP and 15 mM DTT.

La transformation des souches d'Escherichia coli est effectuée selon la méthode décrite par T. Maniatis et coll. (Molecular Cloning Cold Spring Harbor Laboratory Press (1982).

La nucléase "S1" est employée selon la suggestion du fournisseur (Boehringer).

Le remplissage des extrémités cohésives d'ADN pour les rendre franches est réalisé avec l'enzyme "ADN polymérase (large fragment)"(Boehringer) dans un tampon 50 mM NaCl, 10 mM Tris, HCl pH 7,5, 1 mM MgSO$_4$, 1 mM DTT et en présence de quatre deoxynucléotides triphosphates à une concentration de 1 mM.

Les oligonucléotides sont préparés selon la technique décrite par M.J. Gait et coll. dans H.G. Gassen et A. Lang (eds), Chemical and Enzymatic Synthesis of Gene Fragments, Verlag Chemie Press, p.1 et suivantes (1982).

La sonication des bactéries est réalisée avec un Sonicateur Branson (Proscience, France, Modèle B30), après avoir concentré 20 fois les bactéries dans le tampons P.B.S.(0,2 g/l KCl, 0,2 g/l $KH_2PO_4$, 8 g/l NaCl et 1,25 g/l $Na_2HPO_4$). Le temps de sonication est de 6 minutes. La fraction insoluble est solubilisée dans 2,3 % SDS, 5 % béta-mercaptoéthanol, 10 % glycérol et 0,06 M Tris, HCl pH 6,8 et analysée par électrophorèse sur gel d'acrylamide-SDS selon U.K. Laemli, Nature 227, p. 680 et suivantes (1970).

La dénaturation/réduction du t-PA est effectuée par traitement de la fraction insoluble, correspondant à un litre de culture à une densité optique de $1(A_{610})$ avec 5 $cm^3$ d'une solution : 7 M guanidine HCl, 0,1 M béta-mercaptoéthanol et 0,1 M $KH_2PO_4$ pH 7,5 pendant 24 heures à 4°C sous agitation. La concentration de béta-mercaptoéthanol est ensuite abaissée par dialyse contre 1 litre de solution contenant 7 M guanidine HCl, 0,005 M béta-mercaptoéthanol et 0,1 M $KH_2PO_4$ pH 7,5. La renaturation est alors obtenue par dilution (1/100) de cette solution dans un tampon contenant 2,5 M urée, 50 mM Tris HCl pH 8,75, 10 mM NaCl, 5 mM EDTA, 1 mM glutathion réduit, 0,3 mM glutathion oxydé et 10 mM lysine.

Après cette dilution, la solution est incubée à 4°C pendant 24 heures, puis centrifugée. L'activité du t-PA est mesurée après dilution du surnageant (1/100) dans un tampon P.B.S. contenant 0,01 % de Tween 80.

0236209

Conformément aux dispositions du Traité de Budapest, ont été déposés au CBS à Baarn (Pays-Bas), le 3 février 1987 :

- Un échantillon du microorganisme E.coli OR1319 contenant le plasmide pXL 130 (souche G-680) sous le numéro CBS 147-87.
- Un échantillon du microorganisme E.coli B contenant le plasmide pXL 382 (souche G-1514) sous le numéro CBS 148-87.

REVENDICATIONS

1 - Procédé de préparation microbiologique de l'activateur tissulaire humain du plasminogène ou t-PA caractérisé en ce que l'on cultive une bactérie capable d'assurer le maintien stable d'un plasmide contenant un promoteur choisi parmi le promoteur $P_L$ du bactériophage lambda et le promoteur Ptrp de l'opéron tryptophane d'E.coli, le site de fixation des ribosomes du gène cII du bactériophage lambda éventuellement dépourvu de la séquence de terminaison de la transcription tR1, le gène du t-PA humain contenant en 5' un codon d'initiation ATG et, éventuellement, le site de terminaison de la région précoce du bactériophage T7.

2 - Procédé selon la revendication 1 caractérisé en ce que le plasmide contient le promoteur $P_L$ du bactériophage lambda, le site de fixation des ribosomes du gène cII du bactériophage lambda et le gène du t-PA humain contenant en 5' un codon d'initiation ATG.

3 - Procédé selon la revendication 1 caractérisé en ce que le plasmide contient le promoteur Ptrp de l'opéron tryptophane d'E.coli, le site de fixation des ribosomes du gène cII du bactériophage lambda dépourvu de la séquence de terminaison de la transcription tR1, le gène du t-PA humain contenant en 5' un codon d'initiation ATG et le site de terminaison de la région précoce du bactériophage T7.

4 - Procédé de préparation de la forme active de l'activateur tissulaire humain du plasminogène caractérisé en ce que l'on convertit le produit inactif et insoluble obtenu selon l'une des revendications 1, 2 ou 3 en produit actif et soluble en utilisant une méthode de dénaturation et renaturation qui permet un réarrangement des structures secondaire et tertiaire de la chaîne polypeptidique.

5 - Procédé selon l'une des revendications 1, 2 ou 3 caractérisé en ce que la bactérie capable d'assurer le maintien stable du plasmide est choisie parmi les souches d'E.coli.

6 - Procédé selon la revendication 5 caractérisé en ce que l'on utilise une souche d'E.coli contenant le gène "cro" du bactériophage lambda.

7 - Le plasmide "pXL130" caractérisé en ce qu'il contient le promoteur $P_L$ du bactériophage lambda, le site de fixation des ribosomes du gène cII du bactériophage lambda, le codon d'initiation ATG fusionnés avec le gène de structure de l'activateur tissulaire humain du plasminogène.

8 - Le plasmide "pXL382" caractérisé en ce qu'il contient le promoteur Ptrp de l'opéron tryptophane d'E.coli, le site de fixation des ribosomes du gène cII du bactériophage lambda dépourvu du site tR1 de terminaison de la transcription, le codon d'initiation ATG fusionnés avec le gène de structure de l'activateur tissulaire humain du plasminogène et le site de terminaison de la région précoce du bactériophage T7.

9 - L'activateur tissulaire humain du plasminogène lorsqu'il est obtenu par le procédé selon l'une des revendications 1, 2 ou 3.

10 - La forme active de l'activateur tissulaire humain du plasminogène lorsqu'elle est obtenue selon le procédé de la revendication 4.

Clone "cDNA" du t-PA
(2310 paires de bases)

EcoRI     EcoRI   EcoRI     EcoRI

séquence DNA t-PA
séquence DNA vecteur

Figure 1

PL. II/15                    0236209

Séquence du clone "cDNA" du t-PA

```
     10        20        30        40        50        60        70        80
GAATTCCCCAGCCCTGGACTCCTGTGAAGCAATCATGGATGCAATGAAGAGAGGGCTCTGCTGTGTGCTGCTGCTGTGTG
CTTAAGGGGTCGGGACCTGAGGACACTTCGTTAGTACCTACGTTACTTCTCTCCCGAGACGACACACGACGACGACACAC


     90       100       110       120       130       140       150       160
GAGCAGTCTTCGTTTCGCCCAGCCAGGAAATCCATGCCCGATTCAGAAGAGGAGCCAGATCTTACCAAGTGATCTGCAGA
CTCGTCAGAAGCAAAGCGGGTCGGTCCTTTAGGTACGGGCTAAGTCTTCTCCTCGGTCTAGAATGGTTCACTAGACGTCT


    170       180       190       200       210       220       230       240
GATGAAAAAACGCAGATGATATACCACCAACATCAGTCATGGCTGCGCCCTGTGCTCAGAAGCAACCGGGTGGAATATTG
CTACTTTTTTGCGTCTACTATATGGTCGTTGTAGTCAGTACCGACGCGGGACACGAGTCTTCGTTGGCCCACCTTATAAC


    250       260       270       280       290       300       310       320
CTGGTCCAACAGTCGCAGGGCACAGTGCCACTCAGTGCCTGTCAAAAGTTGCAGCGAGCCAAGGTGTTTCAACGGGGGCA
GACCACCTTGTCACCGTCCCGTGTCACCGTGAGTCACGGACAGTTTTCAACGTCGCTCGGTTCCACAAAGTTGCCCCCGT


    330       340       350       360       370       380       390       400
CCTGCCACCAGGCCCTGTACTTCTCAGATTTCGTCTGCCAGTGCCCCGAACGATTTGCTGGGAAGTGCTGTGAAATAGAT
GGACGGTCGTCCGGGACATGAAGAGTCTAAAGCACACGGTCACGGGGCTTCCTAAACGACCCTTCACGACACTTTATCTA


    410       420       430       440       450       460       470       480
ACCAGGGCCACGTGCTACCACGACCAGCGGCATCAGCTACAGCGGCACGTGGAGCACAGCGGAGAGTGGCGCCGAGTGCAC
TGGTCCCGGTGCACGATGCTCCTCGTCCCGTAGTCGATGTCCCCGTGCACCTCGTGTCGCCTCTCACCGCGGCTCACGTG


    490       500       510       520       530       540       550       560
CAACTGGAACAGCAGCCCGTTGGCCCACAAGCCCTACAGCGGGCGGAGGCCAGACGCCATCAGGCTGGGCCTGGGGAACC
GTTGACCTTGTCGTCGGGCAACCGGGTCTTCGGGATGTCGCCCGCCTCCGGTCTGCGGTAGTCCGACCCGGACCCCTTGG
```

Figure 2

```
        570       580       590       600       610       620       630       640
ACAACTACTGCAGAAACCCACATCCACACTCAAAGCCCTGGTCCTACGTCTTTAAGGCCGSCGGAAGTACAGCTCAGAGTTC
TGTTGATCACGTCTTTGCGTCTACCTCTCACTTTCGGGACCACGATGCAGAAATTCCGCCCCTTCATGTCGAGTCTCAAG
```

```
        650       660       670       680       690       700       710       720
TGCAGCACCCCTGCCTCCTCTGACGGCAAACAGTGACTGCTACTTTGGGAATGGGTCAGCCTACCGTGGCACGCACAGCCT
ACGTCGTGGGGACGGACGAGACTCCCTTTGTCACTGACGATGAAACCCTTACCCAGTCGGATGGCACCGTGCGTGTCGGCA
```

```
        730       740       750       760       770       780       790       800
CACCCACTCGGGTGCCTCCTGCCTCCCGTGGAATTCCATGATCCTGATAGGCAAGGTTTACACAGCACAGAACCCCAGTG
GTCGCTCAGCCCACCGACGACGGAGGGCACCTTAAGGTACTAGGACTATCCGTTCCAAATGTGTCGTGTCTTGGGGTCAC
```

```
        810       820       830       840       850       860       870       880
CCCAGGCACTGGGCCTGGGCAAACATAATTACTGCCGGAATCCTGATGGGGATGCCAAGCCCTGGTGCCACGTGCTGAAG
GCGTCCGTGACCCGGACCCGTTTGTATTAATGACGGCCTTAGGACTACCCCTACGGTTCGGGACCACGGTGCACGACTTC
```

```
        890       900       910       920       930       940       950       960
AACCGCAGGCTCACGTGGCAGTACTGTGATGTGCCCTCCTGCTCCACCTGCGGCCTGAGACAGTACAGCCAGCCTCAGTT
TTGGCCTCCCACTGCACCCTCATCACACTACACGGGAGGACGAGGTGGACGCCGGACTCTGTCATGTCGGTCGGAGTCAA
```

```
        970       980       990       1000      1010      1020      1030      1040
TCGCATCAAAGGAGGGCTCTTCGCCGACATCGCCTCCCACCCCTGGCAGGCTGCCATCTTTGCCAAGCACAGGAGGTCGC
ACCCTAGTTTCCTCCCGAGAACCGCCTGTAGCGGAGGGTGGGGACCGTCCGACGGTAGAAACGGTTCGTGTCCTCCAGCG
```

```
        1050      1060      1070      1080      1090      1100      1110      1120
CCGGACAGCCCTTCCTGTGCGCGCGGCATACTCATCAGCTCCTGCTGGATTCTCTCTGCCGCCCACTGCTTCCAGGAGAGG
GCCCTCTCGCCAAGGACACGCCCCCGTATGAGTAGTCGAGGACGACCTAAGAGAGACGGCCGGGTGACGAAGGTCCTCTCC
```

```
        1130      1140      1150      1160      1170      1180      1190      1200
TTTCCCCCCACCACCTGACCGTCATCTTGGGCAGAACATACCGGGTGGTCCCTGCCGAGGAGGAGCAGAAATTTGAAGT
AAACGCCCCGGTGGTGGCACTCCCACTAGAACCCGTCTTGTATGGCCCACCAGGGACCGCTCCTCCTCGTCTTTAAACTTCA
```

Figure 2 (suite 1)

1210      1220      1230      1240      1250      1260      1270      1280

CCAAAAATACATTGTCCATAACCAATTCCATGATCACACTTACGACAATCACATTGCGCTGCTGCAGCTGAAATCGGATT
CCTTTTTATGTAACAGGTATTCCTTAACCTACTACTGTGAATGCTGTTACTGTAACGCGACGACGTCGACTTTAGCCTAA


1290      1300      1310      1320      1330      1340      1350      1360

CCTCCCCCTGTGCCCAGCAGACGACCGTCGTCCGCACTGTGTGCCGTTCCCCCGGCGGACCTGCAGCTGCCGGACTGGACG
GGAGGGGGACACGGGTCCTCTCGTCGCACCAGGCGTGACACACGGAAGCGGGCCGCCTGGACGTCGACGGCCTGACCTGC


1370      1380      1390      1400      1410      1420      .1430      1440

GAGTCTCACCTCTCCCCCTACCGCAACCATGAGGCCTTGTCTCCTTTCTATTCGGAGCGGCTGAAGGAGGCTCATGTCAG
CTCACACTCGAGAGGCCGATGCCGTTCGTACTCCGGAACAGAGGAAAGATAAGCCTCGCCGACTTCCTCCGAGTACAGTC


1450      1460      1470      1480      1490      1500      1510   .   1520

ACTGTACCCATCCAGCCGCTGCACATCACAACATTTACTTAACAGAACAGTCACCGACAACATGCTGTGTGCTGGAGACA
TGACATCGGTAGGTCGGCGACCTGTAGTGTTGTAAATGAATTGTCTTGTCAGTGGCTGTTGTACGACACACGACCTCTGT


1530      1540      1550      1560.     1570      1580      1590      1600

CTCGGACCGGCCGGCCCCAGGCAAACTTGCACGACGCCTGCCAGGGCCGATTCGCGGAGGCCCCCTGGTGTGTCTGAACGAT
GAGCCCTCGCCTCCCCCGGTCCCTTTCAACGTGCTGCGGACGGTCCCGCTAAGCCCTCCGGGGGACCACACAGACTTGCTA


1610      1620      1630      1640      1650      1660      1670      1680

GCCCCCATCACTTTCCTGCCCATCATCAGCTGCCGCCTGGGCTGTGGACAGAAGGATGTCCCCGGGTGTGTACACCAAGGT
CGGGGGTAGTGAAAGGACGGGTAGTAGTCGACCCCCGCACCCGACACCTGTCTTCCTACAGGGCCCACACATGTGGTTCCA


1690      1700      1710      1720      1730      1740      1750      1760

TACCAACTACCTAGACTGGATTCCTGACAACATGCGACCGTGACCAGGAACACCCGACTCCTCAAAAGCAAATGAGATCC
ATGGTTGATGCATCTGACCTAAGGACTGTTGTACGCTGGCACTGGTCCTTGTGGGCTGAGGAGTTTTCGTTTACTCTAGG


1770      1780      1790      1800      1810      1820      1830      1840

CCCCTCTTCTTCTTCAGAAGACACTGCAAAGGCGCAGTGCTTCTCTACAGACTTCTCCAGACCCACCACACCGCAGAAGC
GGGGAGAAGAAGAAGTCTTCTGTGACGTTTCCGCGTCACGAAGAGATGTCTGAAGAGGTCTGGGTGGTGTGGCGTCTTCG


Figure 2 (suite 2)

```
        1850      1860      1870      1880      1890      1900      1910      1920
CGCACCAGACCCTACAGGAGACGCAAGACTCCATTTTCCCAGATACTTCCCATTTTGGAAGTTTTCAGGACTTGGTCTGA
CCCTGCTCTGGCATGTCCTCTCCCTTCTCACGTAAAAGGGTCTATGAAGGGTAAAACCTTCAAAAGTCCTGAACCAGACT


        1930      1940      1950      1960      1970      1980      1990      2000
TTTCACGATACTCTCTCACATGCGAAGACATGAATGCACACTAGCCTCTCCAGGAATGCCTCCTCCCTGGGCAGAAGTGG
AAAGTCCTATCAGACAGTCTACCCTTCTGTACTTACGTGTGATCGGAGAGGTCCTTACGGAGGAGGGACCCGTCTTCACC


        2010      2020      2030      2040      2050      2060      2070      2080
CCATGCCACCCTGTTTTCGCTAAAGCCCAACCTCCTGACCTGTCACCGTGAGCAGCTTTGGAAACAGGACCACAAAAATG
GGTACGGTGCGACAAAACCGATTTCGGGTTGGAGGACTGGACAGTGGCACTCGTCGAAACCTTTGTCCTGGTGTTTTTAC


        2090      2100      2110      2120      2130      2140      2150      2160
AAAGCATGTCTCAATAGTAAAACAAACAAGAGATCTTTCAGGAAAGACGGATTGCATTAGAAATAGACAGTATATTTATA
TTTCGTACACAGTTATCATTTTCTTTGTTCTCTAGAAAGTCCTTTCTGCCTAACGTAATCTTTATCTGTCATATAAATAT


        2170      2180      2190      2200      2210      2220      2230      2240
CTCACAACAGCCACCACGGCCTCAAAGTTGGGGTGAGGCTGGCTGGCCCGTCATGTTCCTCAAAGCGCCGCCCTTGACGTC
CACTGTTCTCGCTCGTCCCGGAGTTTCAACCCCCTCCGACCGACCGGGCAGTACAAGGAGTTTCGCGGCGGGAACTGCAG


        2250      2260      2270      2280      2290      2300      2310      2320
AACTCTCCTTCCCCTTTCCCCACTCCCTGGCTCTCAGAAGGTATTCCTTTTGTGTACAGTGTGGGAATTC
TTCAGAGGAAGGGCAAAGGGGTCAGCGACCGAGAGTCTTCCATAAGGAAAACACATGTCACACCCTTAAG
```

Figure 2 (suite 3)

PL. VI/15

0236209

<u>Traduction du gène du t-PA</u>

MET ASP ALA MET LYS ARG GLY LEU CYS CYS VAL LEU LEU LEU CYS GLY ALA VAL PHE VAL
ATG GAT GCA ATG AAG AGA GGG CTC TGC TGT GTG CTG CTG CTG TGT GGA GCA GTC TTC GTT

SER PRO SER GLN GLU ILE HIS ALA ARG PHE ARG ARG GLY ALA ARG SER TYR GLN VAL ILE
TCG CCC AGC CAG GAA ATC CAT GCC CGA TTC AGA AGA GGA GCC AGA TCT TAC CAA GTG ATC

CYS ARG ASP GLU LYS THR GLN MET ILE TYR GLN GLN HIS GLN SER TRP LEU ARG PRO VAL
TGC AGA GAT GAA AAA ACG CAG ATG ATA TAC CAG CAA CAT CAG TCA TGG CTG CGC CCT GTG

LEU ARG SER ASN ARG VAL GLU TYR CYS TRP CYS ASN SER GLY ARG ALA GLN CYS HIS SER
CTC AGA AGC AAC CGG GTG GAA TAT TGC TGG TGC AAC AGT GGC AGG GCA CAG TGC CAC TCA

VAL PRO VAL LYS SER CYS SER GLU PRO ARG CYS PHE ASN GLY GLY THR CYS GLN GLN ALA
GTG CCT GTC AAA AGT TGC AGC GAG CCA AGG TGT TTC AAC GGG GGC ACC TGC CAG CAG GCC

LEU TYR PHE SER ASP PHE VAL CYS GLN CYS PRO GLU GLY PHE ALA GLY LYS CYS CYS GLU
CTC TAC TTC TCA GAT TTC GTG TGC CAG TGC CCC GAA GGA TTT GCT GGG AAG TGC TGT GAA

ILE ASP THR ARG ALA THR CYS TYR GLU ASP GLN GLY ILE SER TYR ARG GLY THR TRP SER
ATA GAT ACC AGG GCC ACG TGC TAC GAG GAC CAG GGC ATC ACC TAC AGG GGC ACG TGG AGC

Figure 3

455

```
THR ALA GLU SER GLY ALA GLU CYS THR ASN TRP ASN SER SER ALA LEU ALA GLN LYS PRO
ACA GCG GAG AGT GGC GCC GAG TGC ACC AAC TGG AAC AGC AGC GCG TTG GCC CAG AAG CCC
```

515

```
TYR SER GLY ARG ARG PRO ASP ALA ILE ARG LEU GLY LEU GLY ASN HIS ASN TYR CYS ARG
TAC AGC GCG CGG AGG CCA GAC GCC ATC AGG CTG GGC CTG GGG AAC CAC AAC TAC TGC AGA
```

575

```
ASN PRO ASP ARG ASP SER LYS PRO TRP CYS TYR VAL PHE LYS ALA GLY LYS TYR SER SER
AAC CCA GAT CGA GAC TCA AAG CCC TGG TGC TAC GTC TTT AAG GCG GGG AAG TAC AGC TCA
```

635

```
GLU PHE CYS SER THR PRO ALA CYS SER GLU GLY ASN SER ASP CYS TYR PHE GLY ASN GLY
GAG TTC TGC AGC ACC CCT GCC TGC TCT GAG GGA AAC AGT GAC TGC TAC TTT GGG AAT GGG
```

695

```
SER ALA TYR ARG GLY THR HIS SER LEU THR GLU SER GLY ALA SER CYS LEU PRO TRP ASN
TCA GCC TAC CGT GGC ACG CAC AGC CTC ACC GAG TCG GGT GCC TCC TGC CTC CCG TGG AAT
```

755

```
SER MET ILE LEU ILE GLY LYS VAL TYR THR ALA GLN ASN PRO SER ALA GLN ALA LEU GLY
TCC ATG ATC CTG ATA GGC AAG GTT TAC ACA GCA CAG AAC CCC AGT GCC CAG GCA CTG GGC
```

815

```
LEU GLY LYS HIS ASN TYR CYS ARG ASN PRO ASP GLY ASP ALA LYS PRO TRP CYS HIS VAL
CTG GGC AAA CAT AAT TAC TGC CGG AAT CCT GAT GGG GAT GCC AAG CCC TGG TGC CAC GTG
```

875

```
LEU LYS ASN ARG ARG LEU THR TRP GLU TYR CYS ASP VAL PRO SER CYS SER THR CYS GLY
CTC AAG AAC CGC AGG CTC ACG TGG GAG TAC TGT GAT GTG CCC TCC TGC TCC ACC TGC GGC
```

Figure 3 (suite 1)

935

LEU ARG GLN TYR SER GLN PRO GLN PHE ARG ILE LYS GLY GLY LEU PHE ALA ASP ILE ALA
CTC AGA CAG TAC AGC CAG CCT CAG TTT CGC ATC AAA GGA GGG CTC TTC GCC GAC ATC GCC

995

SER HIS PRO TRP GLN ALA ALA ILE PHE ALA LYS HIS ARG ARG SER PRO GLY GLU ARG PHE
TCC CAC CCC TCG CAG GCT GCC ATC TTT GCC AAG CAC AGG AGG TCG CCC GGA GAG CGG TTC

1055

LEU CYS GLY GLY ILE LEU ILE SER SER CYS TRP ILE LEU SER ALA ALA HIS CYS PHE GLN
CTC TGC GGG GGC ATA CTC ATC AGC TCC TGC TGG ATT CTC TCT GCC GCC CAC TGC TTC CAG

1115

GLU ARG PHE PRO PRO HIS HIS LEU THR VAL ILE LEU GLY ARG THR TYR ARG VAL VAL PRO
GAG AGG TTT CCG CCC CAC CAC CTG ACG GTG ATC TTG GGC AGA ACA TAC CGG GTG GTC CCT

1175

GLY GLU GLU GLU GLN LYS PHE GLU VAL GLU LYS TYR ILE VAL HIS LYS GLU PHE ASP ASP
GCC GAG GAG GAG CAG AAA TTT GAA GTC GAA AAA TAC ATT GTC CAT AAG GAA TTC GAT GAT

1235

ASP THR TYR ASP ASN ASP ILE ALA LEU LEU GLN LEU LYS SER ASP SER SER ARG CYS ALA
GAC ACT TAC GAC AAT GAC ATT GCG CTG CTG CAG CTG AAA TCG GAT TCG TCC CGC TGT GCC

1295

GLN GLU SER SER VAL VAL ARG THR VAL CYS LEU PRO PRO ALA ASP LEU GLN LEU PRO ASP
CAG GAG AGC AGC GTG GTC CGC ACT GTG TGC CTT CCC CCG GCG GAC CTG CAG CTG CCG GAC

1355

TRP THR GLU CYS GLU LEU SER GLY TYR GLY LYS HIS GLU ALA LEU SER PRO PHE TYR SER
TCC ACG GAG TGT GAG CTC TCC GGC TAC GGC AAG CAT GAG GCC TTG TCT CCT TTC TAT TCG

Figure 3 (suite 2)

1415

GLU ARG LEU LYS GLU ALA HIS VAL ARG LEU TYR PRO SER SER ARG CYS THR SER GLN HIS
GAG CGG CTG AAG GAG GCT CAT GTC AGA CTG TAC CCA TCC AGC CGC TGC ACA TCA CAA CAT

1475

LEU LEU ASN ARG THR VAL THR ASP ASN MET LEU CYS ALA GLY ASP THR ARG SER GLY GLY
TTA CTT AAC AGA ACA GTC ACC GAC AAC ATG CTG TGT GCT GGA GAC ACT CGG AGC GGC GGG

1535

PRO GLN ALA ASN LEU HIS ASP ALA CYS GLN GLY ASP SER GLY GLY PRO LEU VAL CYS LEU
CCC CAG GCA AAC TTG CAC GAC GCC TGC CAG GGC GAT TCG GGA GGC CCC CTG GTG TGT CTG

1595

ASN ASP GLY ARG MET THR LEU VAL GLY ILE ILE SER TRP GLY LEU GLY CYS GLY GLN LYS
AAC GAT GGC CGC ATG ACT TTG GTG GGC ATC ATC AGC TGG GGC CTG GGC TGT GGA CAG AAG

1655

ASP VAL PRO GLY VAL TYR THR LYS VAL THR ASN TYR LEU ASP TRP ILE ARG ASP ASN MET
GAT GTC CCG GGT GTG TAC ACC AAG GTT ACC AAC TAC CTA GAC TGG ATT CGT GAC AAC ATG

1715

ARG PRO ⁂ CCAGGAACACCCGACTCCTCAAAAGCAAATGAGATCCCGCCTCTTCTTCTTCAGAAGACACTGCAAA
CCA CCG TGA

Figure 3 (suite 3)

PL. X/15

0236209

RI - Bam = 578 paires de bases

Figure 4

Figure 5

Figure 5 (suite)

Figure 6

Stimulation par la fibrine

Figure 7

$^A420$

PL. XIV/15

0236209

Inhibition de l'activité par des anticorps anti-tPA

Figure 8

## DOCUMENTS CONSIDERES COMME PERTINENTS

| Catégorie | Citation du document avec indication. en cas de besoin. des parties pertinentes | Revendication concernée | CLASSEMENT DE LA DEMANDE Int Cl.4 |
|---|---|---|---|
| Y | EP-A-0 093 619 (GENETECH) <br> * Revendications * | 1 | C 12 N 15/00 <br> C 12 N 9/64 |
| | --- | | |
| Y | EP-A-0 159 123 (ELI LILY) <br> * Pages 88-89 * | 1 | |
| | --- | | |
| Y | EP-A-0 114 777 (TRANSGENE) <br> * Revendications; figures * | 1 | |
| | --- | | |
| Y | EP-A-0 137 710 (GENEX) <br> * Revendications * | 1 | |
| | ----- | | |

**DOMAINES TECHNIQUES RECHERCHES (Int. Cl.4)**

C 12 N

Le présent rapport de recherche a été établi pour toutes les revendications

| Lieu de la recherche | Date d'achèvement de la recherche | Examinateur |
|---|---|---|
| LA HAYE | 02-06-1987 | DELANGHE L.L.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur. mais publié à la date de dépôt ou après cette date
D : cité dans la demande
L : cité pour d'autres raisons

& : membre de la même famille. document correspondant

OEB Form 1503 03 82